# EUROPÄISCHE PATENTSCHRIFT

(11) **EP 0 843 690 B1**
(45) Veröffentlichungstag und Bekanntmachung des Hinweises auf die Patenterteilung: **20.02.2002**
(21) Anmeldenummer: 96926375.5
(22) Anmeldetag: 19.07.1996
(51) Int. Cl.: C08F 220/04, A61L 15/60

(54) **ABSORPTIONSMITTEL FÜR WASSER UND WÄSSRIGE FLÜSSIGKEITEN SOWIE VERFAHREN ZU IHRER HERSTELLUNG UND VERWENDUNG**
ABSORBING AGENTS FOR WATER AND AQUEOUS LIQUIDS AND PROCESS FOR THEIR PRODUCTION AND USE
AGENTS ABSORBANT L'EAU ET LES LIQUIDES AQUEUX, LEUR PROCEDE DE FABRICATION ET LEUR UTILISATION

(30) Priorität: 09.08.1995 DE 19529348
(43) Veröffentlichungstag der Anmeldung: 27.05.1998
(73) Patentinhaber: STOCKHAUSEN GmbH & Co. KG, 47805 Krefeld (DE)
(72) Erfinder: DAHMEN, Kurt, D-41239 Mönchengladbach (DE); PEPPMÖLLER, Reinmar, D-47802 Krefeld (DE)
(74) Vertreter: Kahlhöfer, Hermann, Dipl.-Phys.
(86) Internationale Anmeldenummer: EP9603203
(87) Internationale Veröffentlichungsnummer: WO9706190

(56) Entgegenhaltungen:
- EP-A- 0 315 185
- EP-A- 0 467 073
- WO-A-91/18031
- WO-A-95/11932
- DE-A- 4 418 818

## Beschreibung

Die Erfindung betrifft wasserunlösliche, wasserquellbare Polymerisate mit hohem Aufnahmevermögen für Wasser und wässrige Flüssigkeiten auf der Basis von vernetzten, hydrophilen Homo- und Copolymerisaten sowie Pfropfpolymerisaten aus ethylenisch ungesättigten, Säuregruppen enthaltenden, polymerisierbaren Monomeren und das Herstellungsverfahren sowie die Verwendung dieser Polymerisate.

Polymerisate, die große Mengen von wässrigen Flüssigkeiten, insbesondere Körperflüssigkeiten wie Urin aufnehmen, sind als superabsorbierende Polymere bekannt.

Die Herstellung der Absorptionsmittel erfolgt durch radikalische Polymerisation unter vorzugsweiser Verwendung von monoethylenisch ungesättigten Carbonsäuren, wie Acrylsäure und deren Alkalisalze, in wässriger Lösung oder nach den Verfahren der inversen Suspensions- oder Emulsionspolymerisation, wie sie in US 4,286,082, DE-PS 27 06 135, US 4,340,706, DE-PS 37 13 601 und DE-PS 28 40 010 beschrieben werden.

Durch die Auswahl der Monomerenzusammensetzung, der Vernetzer sowie der Polymerisationsbedingungen und der Verarbeitungsbedingungen für das Polymerisatgel lassen sich Polymerisate mit unterschiedlichen Absorbereigenschaften herstellen. Weitere Möglichkeiten bietet die Herstellung von Pfropfpolymerisaten, beispielsweise unter Verwendung chemisch modifizierter Stärke, Cellulose und Polyvinylalkohol nach DE-OS 26 12 846 und die Nachbehandlung der Polymerisatgele oder der pulverförmigen Harze durch Nachvernetzung der Oberflächen der Polymerisatpartikel, beispielsweise nach DE 40 20 780 C1.

Für die Verwendung der Polymerisate in Hygiene und Sanitärbereich werden Polymerisate erzeugt, deren Neutralisationsgrad zwischen 50 und 80 Mol%, bezogen auf die polymerisierten, Säuregruppen enthaltenden Monomereinheiten, beträgt, so daß bei der Verwendung hautneutral wirkende Hydrogele gebildet werden.

Die Einstellung des Neutralisationsgrades kann auf unterschiedliche Weise erfolgen, wobei die Neutralisation oder teilweise Neutralisation der sauren Monomeren häufig vor der Polymerisation vorgenommen wird. Aber auch die Neutralisation oder teilweise Neutralisation von saure, polymerisierte Monomereinheiten enthaltendem Hydrogel ist bekannt.

Nach EP 205 674 A1 werden vorteilhaft völlig saure Polymerisate bei Temperaturen im Bereich von 0 bis 100 °C, vorzugsweise 5 bis 40 °C hergestellt, die durch nachträgliche Teilneutralisation des Hydrogels eingestellt werden. Die Polymeriste zeichnen sich durch eine verbesserte Gelstärke und Absorptionskapazität sowie durch einen geringen wasserlöslichen Polymerisatanteil aus.

Nach US 5,145,906 und EP 530 438 B1 werden ohne Neutralisation der Monomeren aus Acrylsäure zusammen mit wasserlöslichen Hydroxylgruppen enthaltenden Polymeren bei Temperaturen im Bereich von 5 bis 20 °C unter adiabatischen Bedingungen Polymerisatgele hergestellt, die anschließend zerteilt und durch wässrige Basen teilweise oder vollständig neutralisiert und dann in Gegenwart eines Nachvernetzungsmittels erneut zerkleinert und einer Wämebehandlung unterworfen werden.

Die genannten Verfahren sind nachteilig, da die Polymerisation der Monomerenlösung, wie EP 467 073 A1 zeigt, sehr langsam verläuft, so daß die Polymerisation nur diskontinuierlich im Batchverfahren betrieben werden kann. Die Erhöhung der Initiatormenge oder eine erhöhte Reaktionstemperatur wirken sich nachteilig auf die gewünschten Polymerisateigenschaften aus. Weiterhin bereitet es Schwierigkeiten, das vollständig saure Polymerisatgel zu zerteilen und die Neutralisation erfolgt wegen des diffusionsbestimmten Vorgangs langsam, wobei im Oberflächenbereich der Polymerisatpartikel ein Basenüberschuß verbleibt, während noch reaktionsfähige polymerisierte Säuregruppe enthaltende Monomereinheiten im Gelinneren vorliegen, wo sie jedoch für die Nachvernetzung im Oberflächenbereich nicht verfügbar sind.

In EP 467 073 A1 werden daher Polymerisate beschrieben, die bei einer kontrollierten Polymerisationstemperatur im Bereich von 20 bis 70 °C, inbesondere bei 55 bis 60 °C innerhalb von 5 Std. erhalten werden. Nach dem beschriebenen Verfahren erfolgt dann eine Neutralisation der Polyacrylsäure bis zu einem Neutralisationsgrad von 72 %. Das getrocknete und gemahlene Polymerisat wird anschließend der Nachvernetzung zugeführt. Die nach diesem Verfahren erhaltenen Polymerisate weisen jedoch nur eine Begrenzte Flüssigkeitsabsorption bei der Aufnahme von Flüssigkeiten unter Druckbelastung auf. Weiterhin weisen die bekannten Polymerisate nach der Flüssigkeitsabsorption ein erhebliches Rückfeuchtungsverhalten auf, das bei der Anwendung, insbesondere im Hygienebereich nachteilig ist.

Es besteht daher die Aufgabe, Polymerisate mit verbesserten Absorptionseigenschaften, insbesondere mit verbesserter Flüssigkeitsaufnahme unter Druckbelastung und verbesserten Rewet-Verhalten und ein Verfahren zu ihrer Herstellung unter Verkürzung der Polymerisationszeit und unter Verbesserung der Nachvernetzung bereitzustellen.

Diese Aufgabe wird gelöst durch ein pulverförmiges, Wasser- und wässrige Flüssigkeiten absorbierendes, vernetztes Polymerisat, gebildet aus a) 55 - 99,9 Gew. % mindestens eines polymerisierten, ungesättigten, polymerisierbaren, Säuregruppen enthaltenden Monomeren, das zu mindestens 50 Mol% neutralisiert als Salz vorliegt, b) 0 - 40 Gew.% polymerisiertem, ungesättigten, mit dem Monomeren nach a) copolymerisierbaren Monomeren, c) 0,01 - 5,0 Gew.% mindestens eines Vernetzungsmittels und d) 0 - 30 Gew.% eines wasserlöslichen Polymeren, wobei die Summe der Komponenten a) - d) 100 Gew.% ergibt, das dadurch gekennzeichnet ist, daß das Polymerisat aus zu 5 - 30 Mol%, bezogen auf den Säuregruppen enthaltenen Monomerenanteil, teilneutralisierten sauren Polymerisatgelen gebildet wird und
- eine Retention für eine 0,9 %ige wässrige NaCl-Lösung von mindestens 25 g/g Polymerisat,
- eine Flüssigkeitsaufnahme unter einem Druck von 50 g/cm² von mindestens 25 g/g Polymerisat,
- einen Quelldruck nach 20 Minuten von mindestens 700 g, bezogen auf 1 g Polymerisat,
- einen löslichen Anteil nach 16 Std. von höchstens 3,5-10 Gew.%, und
- einen Rewet von höchstens 2,0 g aufweist.

Die Aufgabe wird weiter gelöst durch ein Verfahren zur Herstellung dieses Polymerisats, dadurch gekennzeichnet, daß eine wässrige Lösung mit einem Gehalt von höchstens 30 Gew.% Monomere, bezogen auf die gesamte Lösung, hergestellt wird, die ungesättigten, polymerisierbaren Säuregruppen enthaltenden Monomere vor der Polymerisation mit Basen zu 5,0 - 30 Mol%, vorzugsweise zu 10 - 20 Mol% neutralisiert werden, die Polymerisation unter radikalischen Bedingungen bei einer Temperatur im Bereich von 5 - 30 °C vorzugsweise 8 - 15 °C durch chemische Initiatoren und/oder durch UV-Bestrahlung ausgelöst und adiabatisch fortgeführt wird, das Polymerisatgel nach der Grobzerkleinerung einer Feinzerkleinerung unterworfen wird und mit Basen so neutralisiert wird, daß das Polymerisat einen Gehalt von polymerisierten, neutralisierten Säuregruppen enthaltenden Monomereneinheiten von mindestens 50 Mol% enthält, eine Trocknung des Polymerisatgels auf einen Wassergehalt ≤ 10 Gew.% erfolgt und das getrocknete und gemahlene Polymerisat mit mindestens einem zweifach oder mehrfachfunktionellen, mit Säuregruppen reaktionsfähigen Vernetzungsmitteln bei einer Temperatur im Bereich von 140 - 240 °C zur Reaktion gebracht wird.

Überraschenderweise wurde festgestellt, daß Polymerisate mit hohem Retentionswert und hohem Aufnahmevermögen unter konstantem oder ansteigendem Druck sowie mit geringen löslichen Polymerisatanteilen erhalten werden, wenn die Polymerisation statt mit der vollständig sauren Polymerenlösung mit einer Lösung durchgeführt wird, deren Säuregruppen enthaltende Monomere in geringer Menge durch die Zugabe von Basen neutralisiert worden sind. Der Bereich der Teilneutralisation liegt bei 5 - 30 Mol%, vorzugsweise 5 - 20 Mol% und besonders bevorzugt 5 - 10 Mol%, bezogen auf die Säuregruppen enthaltenden Monomere.

Als polymerisierbare ungesättigte Säuregruppen enthaltende Monomeren a) werden erfindungsgemäß wasserlösliche monoethylenisch ungesättigte Mono- und Dicarbonsäuren, wie Acrylsäure, Methacrylsäure, Ethacrylsäure, Crotonsäure, Sorbinsäure und Maleinsäure, Fumarsäure, Itaconsäure sowie Vinylsulfonsäure, Acrylamido- und/oder Methacrylamido-alkylsulfonsäuren, wie 2-Acrylamido-2-methylpropansulfonsäure, 2-Methacryloyloxiethansulfonsäure, 4-Vinylbenzolsulfonsäure, Allylsulfonsäure, Vinyltoluolsulfonsäure, Vinylphosphonsäure und Vinylbenzolphosphonsäure eingesetzt.

Die bevorzugte wasserlösliche ungesättigte Carbonsäure ist Acrylsäure. Der Anteil anderer ungesättigter Carbonsäuren neben Acrylsäure im Polymerisat kann bis zu 50 Gew.% betragen.

Weiterhin werden als Monomere b) wasserlösliche monoethylenisch ungesättigte Monomere Acrylamid, Methacrylamid, N-alkylierte (Meth)acrylamide, N-Methylol(meth)acrylamid, N-Vinylamide, N-Vinylformamid, N-Vinylacetamid und N-Vinyl-N-Methylacetamid, N-Vinyl-N-Methylformamid, Vinylpyrrolidon sowie Hydroxyalkyl(meth)acrylate, wie Hydroxyethylacrylat und (Meth)acrylsäureester von Polyethylenglycolmonoallylether und Allylether von Polyethylenglycolen verwendet.

Ebenso werden als Monomere b) mit geringer Löslichkeit in Wasser Acrylsäure- und Methacrylsäureester, wie Ethylacrylat und Methylacrylat, Vinylacetat und Styrol in begrenzter Menge eingesetzt. Der Anteil dieser schwer oder begrenzt wasserlöslichen Monomere beträgt maximal 10 Gew.%, bezogen auf die Summe aller Monomeren.

Die genannten Monomeren werden zur Herstellung von Homo- oder Copolymerisaten mit mindestens zwei Monomeren in beliebiger Kombination eingesetzt. Die Monomerenmischung kann weiterhin wasserlösliche Polymere d) mit einem Gehalt von 0-30 Gew.%, bezogen auf die Komponenten der Monomerenlösung enthalten. Als wasserlösliches Polymeres kann ein synthetisches Polymer oder Copolymer und/oder eine natürliches Polymer und/oder ein Derivat eines natürlichen Polymers eingesetzt werden. Beispiele hierfür sind wasserlösliche Homo- oder Copolymerisate der zuvor genannten Monomeren, wie Polyacrylsäure, teilverseiftes Polyvinylacetat, Polyvinylalkohol, Polyalkylenglykol, Stärke, Stärkederivate, pfropfpolymerisierte Stärke, Cellulose und Cellulosederivate, wie Caboxymethylcellulose, Hydroxymethylcellulose sowie Galaktomannane und dessen oxalkylierte Derivat.

Die wässrige Monomerenlösung enthält mindestens einen Vernetzer c) mit einem Anteil von 0,01 - 5,0 Gew.%, vorzugsweise 0,1 - 2,0 Gew.%, bezogen auf alle Anteile der Komponenten a), b) und c) der Monomerenlösung. Als Vernetzer können alle Verbindungen verwendet werden, die mindestens zwei ethylenisch ungesättigte Doppelverbindungen oder eine ethylenisch ungesättigte Doppelbindung und eine gegenüber Säuregruppen reaktive funktionelle Gruppe oder mehrere gegenüber Säuregruppen reaktive funktionelle Gruppe enthalten. Beispiele hierfür sind: Methylenbisacrylamid, Acrylate und Methacrylate von Polyolen wie Butandioldiacrylat, Hexandiol-dimethacrylat, Polyethylenglycol-diacrylat und Trimethylolpropantriacrylat und/oder die Acrylate und Methacrylate der oxalkylierten genannten Polyole, wie des oxalkylierten Trimethylolpropans und des oxalkylierten Pentaerythrits. Vernetzer diese Typs sind unter den Handelbezeichnungen Sartomer® und Craynor® (Fa. Crayvalley Kunstharze GmbH, DE-47918 Tönisvorst) bekannt, von denen insbesondere Sartomer 415, Sartomer 454, Sartomer 494, Sartomer 610 und Craynor 435 verwendbar sind, weiterhin Di- und Polyester von Polyolen und oxethylierten Polyolen mit ungesättigten Monocarbonsäuren und/oder Polycarbonsäuren, wie (Meth)acrylsäureestern von 1,2-Propylenglycolpentaerythrit, Glycerin und Polyglycerin sowie Monoester ungesättigter Alkohole und ethoxylierter ungesättigter Alkohole mit ungesättigten Monocarbonsäuren und/oder Monocarbonsäuren wie Allylacrylat und -methacrylat, Monoallylmaleinat, Allylpolyethylenglycoletheracrylat und -methacrylat, Allylitaconat, Allylpolyethylenglycolether-itaconat und Monoallylpolyethylenglycolether-maleinat, weiterhin Diallylacrylamid, Diallylphthalat, Diallyladipat, Triallylcitrat und Trimonoallylpolyethylenglycolethercitrat, weiterhin Allylether von Di-und Polyolen und deren Oxethylate, wie die Diallylether von Ethylenglycol, Diethylenglycol, Polyethylenglycol, die Triallylether von Glycerin, oxethyliertem Glycerin, Trimethylolpropan und oxethyliertem Trimethylolpropan, die Tetraallylether von Pentaerythrit und oxethyliertem Pentaerythrit sowie Tetraallyloxiethan sowie Polyglycidylether, wie z. B. Ethylenglycoldiglycidether und Glyceringlycidylether. Weiterhin Amine und/oder deren Salze und Amide mit mindestens zwei ethylenisch ungesättigten Alkylgruppen, wie Di- und Triallylamin und Tetraallylammoniumchlorid.

Zur Initierung der radikalischen Polymerisation werden die gebräuchlichen Initiatoren verwendet, z. B. Peroxo- und Azoverbindungen, vorzugsweise in Wasser lösliche und/oder dissoziierende Peroxo- und Azoverbindungen, wie tert. Butylhydroperoxid und 2,2'-Azobis(2-methylpropionamidin)-dihydrochlorid, sowie Redoxsysteme gebildet aus Natrium- und Kaliumperoxomonosulfat, Natrium- und Kaliumperoxidisulfat und Wasserstoffperoxid mit Natrium- und Kaliumsulfit, Natrium- und Kaliumformamidinsulfinat und Ascorbinsäure.

Bei Verwendung der Redoxsysteme wird vorzugsweise das Oxydationsmittel vorgelegt und das Reduktionsmittel danach zugegeben. Insbesondere bei kontinuierlicher Polymerisation erfolgt die Initiierung durch Photokatalyse mit UV-Licht und den bekannten Sensibilisatoren.

Die Neutralisation der Säuregruppen enthaltenden Monomeren erfolgt vorzugsweise vor der Zugabe der anderen Komponenten der Monomerenlösung sowie bevorzugt unter Vorlage der Base. Geeignete Basen sind die Alkalihydroxide, Ammoniak und die aliphatischen primären und sekundären Amine sowie Alkalicarbonate und Alkalihydrogencarbonate. Bevorzugt werden die Alkalihydroxyde Natriumhydroxid und Kalihydroxid sowie Ammoniak und Soda.

Die Monomerenlösung wird vor der Polymerisation auf eine Temperatur im Bereich von 5 - 30 °C, vorzugsweise 8 - 20 °C abgekühlt. Nach der Initiierung erfolgt die Polymerisation im Gegensatz zu den bekannten Arbeitsweisen überraschenderweise ohne inhibierende Effekte, das heißt ohne zeitliche Verzögerung sowohl in der Startphase als auch im weiteren Verlauf der Polymerisation. Die Polymerisation wird im diskontinuierlichen Batchverfahren oder vorteilhaft in kontinuierlicher Weise, z. B. auf dem Bandreaktor vorgenommen.

Das gebildetete Polymergel wird anschließend einer Grob- und Feinzerteilung mittels üblicher Reiß- und/oder Schneidwerkzeuge unterworfen. Die Feinzerteilung erfolgt vorzugsweise mit Hilfe eines Schneidextruders über endständige Lochscheiben, deren Öffnungen Durchmesser im Bereich von 2 - 20 mm, vorzugsweise 5 - 15 mm und besonders bevorzugt 8 - 13 mm aufweisen.

Die nachträgliche Neutralisation des zerkleinerten Polymeristgels erfolgt mit den bei der Teilneutralisation angegebenen Basen, wobei wiederum vorzugsweise Natronlauge, Kaliumlauge und/oder Ammoniak oder Soda, z. B. als Na₂CO₃ ^{·}10 H₂O oder als wässrige Lösung eingesetzt werden. Die Neutralisation erfolgt in einfachen Mischaggregaten z. B. in einer rotierenden Trommel oder in einem Draismischer, wobei die wässrige Lösung der Basen, beispielsweise über Düsen oder Sprühlanzen eingetragen wird. Die Neutralisation erfolgt, bis mindestens 50 Mol%, vorzugsweise 60 - 80 Mol% der Säuregruppen enthaltenden, polymerisierten Monomereinheiten als Salze vorliegen. Die nachträgliche Neutralisation kann auch während der Feinzerteilung des Gels, beispielsweise bei der Zerteilung im Extruder erfolgen.

Durch die geringe Teilneutralisation der Monomerenlauge wird die Affinität des Polymerisatgels gegenüber der Base, vorzugsweise Alkalilauge so verbessert, daß in der Regel ein einfaches Mischaggregat zur Durchführung der weiteren Neutralisation in einem kurzen Zeitraum ausreichend ist, wodurch eine mechanische Schädigung des Polymergels weitgehend vermieden wird.

Die Trocknung des Polymerisatgels erfolgt bis zu einem Wassergehalt im Bereich von 5 - 20 Gew.%, vorzugsweise von ≤ 10 Gew.% bei Temperaturen im Bereich von 100 -190°C. Anschließend wird das Trockengut auf eine Korngröße im Bereich von 20 - 3000µm, vorzugsweise 150 - 850µm zu Polymerisatpulver gemahlen. Die Nachvernetzung des Polymerisats erfolgt auf der Oberfläche der getrockneten Polymerpartikel mit mindestens einem zwei- oder mehrfach funktionellen, mit Säuregruppen, vorzugsweise Carboxylgruppen, reagierenden Vernetzungsmittel, das vorzugsweise in Form einer wasserhaltigen Lösung aufgebracht wird. Als Nachvernetzungsmittel sind Polyole wie Ethylenglycol, 1,2-Propylenglycol, 1,4-Butandiol, Glycerin, Di- und Polyglycerin, Pentaerythrit, die Oxethylate dieser Polyole sowie deren Ester mit Carbonsäure oder der Kohlensäure geeignet. Der Zusatz eines Veresterungskatalysators, z. B. p-Toluolsulfonsäure oder Phosphorsäure ist vorteilhaft. Weiter geeignete Vernetzungmittel sind Di- und Polyglycidylether von Polyolen und Polyethylenglycolen. Solche Verbindungen sind unter dem Handelsnamen Denacol (Nagase (Europe) GmbH, Düsseldorf) kommerziell erhältlich.

Die Nachvernetzung wird bei Temperaturen im Bereich von 150 - 250 °C, vorzugsweise 150 - 200 °C in einem Mischaggregat, beispielsweise in einem Naramischer vorgenommen.

Das erfindungsgemäße Herstellungsverfahren führt zu Polymerisaten, die sich durch deutlich verbesserte Eigenschaften gegenüber den herkömmlichen Produkten auszeichnen. Sie besitzen eine Retention in 0,9 %iger wässriger NaCl-Lösung von mindestens 25 g/g Polymerisat, vorzusweise von 30 g/g, besonders bevorzugt von mindestens 33 g/g Polymerisat. Sie erreichen unter einer Druckbelastung von 50 g/cm² eine Flüssigkeitsaufnahme von mindestens 25 g/g Polymerisat, vorzugsweise mindestens 27 g/g Polymerisat. Die durch Absorption von Wasser oder wässrigen Flüssigkeiten gequollenen erfindungsgemäßen Polymerisate besitzen im gequollenen Zustand, auch unter Druckbelastung, eine hohe Permeabilität für diese Flüssigkeiten, so daß bei nacheinander erfolgendem mehrfachen Flüssigkeitsangebot eine gute Verteilung der Flüssigkeiten unter vollständiger Ausnutzung des Aufnahmevermögens der Polymerisate erreicht wird. Weiterhin geben sie nur in sehr begrenztem Maße die absorbierte wässrige Flüssigkeit ab, so daß der Rewet-Wert höchstens 2,0 g, vorzugsweise höchstens 1,0 g beträgt. Ihre Fähigkeit zur Flüssigkeitsaufnahme wir weiterhin durch einen sehr hohen Quelldruck nach der Methode von Stevens LFRA Texture Analyser von mindestens 700 g, vorzugsweise mindestens 850g, besonders bevorzugt von 900 g, ganz besonders bevorzugt mindestens 1000 g gekennzeichnet. Die erfindungsgemäßen Polymerisate besitzen einen Gehalt an löslichen Polymerisatanteilen von unter 10 Gew.%, vorzugsweise unter 5 Gew.% und besonders bevorzugt unter 3,5 Gew.%.

Die nach dem erfindungsgemäßen Verfahren erhaltenen Absorptionsmittel können mit weiteren Substanzen abgemischt werden. Solche Substanzen sind beispielsweise Füllmittel, wie Kreide, Bentonit oder Kieselgur sowie Fasermaterialien, wie Hanf-, Viskose- oder Cellulosefaser. Auch Farbpigmente, UV-Absorber, Antioxidantien und Agrochemikalien, wie Düngemittel, Herbizide, Fungizide und Insektizide sind als Komponenten einsetzbar. Die erfindungsgemäßen Polymerisate können in den verschiedenen Bereichen eingesetzt werde, vorzugsweise in Hygieneartikeln, wie Babywindeln, Monatsbinden, Wundpflastern und Inkontinenzbeuteln, die der schnellen und vollständigen Absorption von Urin, Menstruationsblut und Wundsekret dienen. Dabei liegen die Polymerisate in Windeln mit einem Gewichtsanteil bezogen auf die Gewichtsmenge von Polymerisat und Fluff von 15 - 100 Gew.%, vorzugsweise 35 - 100 Gew.% und besonders bevorzugt von 30 - 70 Gew.% vor. Weiterhin können als Komponente in Verpackungsbeilagen zur Aufnahme von aus Lebensmitteln freiwerdenden Flüssigkeiten, in Kabelfüllmassen zur Verhinderung des Wassereinbruchs bei Seewasserkabeln als Komponente in strom- und lichtleitenden Kabeln und in hort- und agrikulturellen Substraten, die als Bodenverbesserungsmittel oder als Depotmaterialien zur kontrollierten Abgabe von Wirkstoffen eingesetzt werden oder als Komponente in künstlichem Boden zur Pflanzenzüchtung verwendet werden. Die Erfindung wird durch die nachfolgenden Beispiele belegt, in denen die folgenden Prüfverfahren verwendet werden:

### Retention (TB):

Es werden 200 mg Superabsorber in einen Teebeutel eingewogen und in eine physiologische Kochsalzlösung (0,9 Gew.-% NaCl) 30 min. eingetaucht. Danach wird der Teebeutel zur Entfernung des ungebundenen Wassers 10 min. an den Ecken aufgehängt und dann 5 min. in einer Zentrifuge bei ca. 1400Upm geschleudert. Das resultierende Gewicht wird um den Blindwert und die Einwaage vermindert und auf 1 g umgerechnet (TB g/g).

### Absorption unter Druck (AAP):

Es werden 0,9 g Superabsorber gleichmäßig in eine Zylinder mit einem Innendurchmesser von 6 cm gegeben, dessen Boden aus einem Siebgewebe mit der Maschenweite 35 µm besteht. Auf den Superabsorber drückt ein Gewicht mit wahlweise 20 g/cm³ (AAP_{0,3psi}) oder 50 g/cm³ (AAP_{0,7psi}). Die Zylindereinheit wird gewogen und anschließend auf eine keramische Filterplatte gestellt, die niveaugleich in physiologische Kochsalzlösung eintaucht. Nach 1 Stunde wird die Zylindereinheit zurückgewogen. Als Absorptionswert gilt der Quotient aus der Gewichtsdifferenz der Zylindereinheiten und der Einwaage des Superabsorbers [(G_{nachher} - Gᵥₒᵣₕₑᵣ)/Einwaage].

### Rewet

Der Rewet-Test erfolgt nach den Angaben in EP 0 631 768 A1, S. 19, Z. 39 bis S. 20, Z. 4, wobei anstelle des synthetischen Urins eine 0,9%ige, wässrige NaCl-Lösung verwendet wird.

### Quelldruck (QD):

Die Bestimmung des Quelldrucks Q erfolgt mit Hilfe des Stevens L.F.R.A. Texture Analyser, C. Stevens & Son Ltd., Laboratory Division, St. Albans AL1 1 Ex Hertfordshire, England.
Es werden 0,500 g Superabsorber der Fraktion 20 - 50 mesh in den dazugehörigen Meßzylinder mit 2,7 cm Durchmesser eingewogen und mit 10 ml 0,9%iger NaCl-Lösung versetzt. Danach wird der Meßzylinder mit Hilfe eines Laborboys so weit hochgefahren, bis der Stand der Unterkante des zylindrischen Meßkörpers von der Oberfläche der sich im Meßzylinder befindlichen probe 12 mm beträgt. Durch die Ausdehnung des Gels wird der Meßzylinder nach oben gegen eine Zwei-Weg-Kraftmeßzelle gedrückt und am Gerät in Gramm angezeigt.

### Restmonomer (RM):

Die Bestimmung der Restmengen von Acrylsäure im Superabsorber erfolgt nach Extraktion mit physiologischer Kochsalzlösung bis zum Gleichgewichtszustand gaschromatographisch.

### Lösliche Anteile (LA):

Der innerhalb von 16 Stunden extrahierbare, carbonsäurebezogene Polymeranteil in einem Superabsorber wird nach einer Methode bestimmt, die in Eu.P.Appl. 0 205 674, S. 34 - 37 beschrieben ist, mit dem Unterschied, daß an Stelle des synthetischen Urins physiologische Kochsalzlösung benutzt wird. Die Mengenangabe erfolgt in Gew.% bezogen auf das getrocknete, gemahlene Polymerisat.

Die Erfindung wird anhand der folgenden Beispiele und Vergleichsdaten erläutert:

### Beispiel 1:

80 g Acrylsäure und 0,24 g eines Triacrylates von einem oxethylierten (15 Mole Ethylenoxid) Trimethylolpropan werden in 244 g Wasser gelöst und mit 4,4 g 50 %-iger Natronlauge vorneutralisiert. Nach Kühlung mit Eis auf 6 bis 8 °C und Spülung mit Stickstoff bis auf einen restlichen Sauerstoffgehalt von max. 0,3 ppm werden 0,033 g 2,2'-Azobis(2-methyl-propionamidin)-dihydrochlorid, 0,12 g Natriumpersulfat und 0,023 g Wasserstoffperoxid (35 %) eingetragen. Dann wird die Polymerisation mit 0,003 g Ascorbinsäure gestartet.
Nach einer Initiierungsphase von ca. 5 min steigt die Temperatur stetig an und erreicht nach ca.20 min. die Maximaltemperatur von 72°C. Für die Nachreaktion werden weitere 15 min. eingehalten und sodann das entstandene Polymergel durch einen Fleischwolf gegeben. Die zerkleinerte Masse wird anschließend in einer Trommel mit horizontaler Rührwelle mit 57,8 g 50 %-iger Natronlauge vermischt (Erhöhung des Neutralisationsgrades auf 70 %), bei 140 bis 160°C auf einen Wassergehalt von <10 % getrocknet und auf eine Partikelgröße von >180 bis <850 µm gemahlen. Das erhaltene Granulat wird mit Ethylencarbonat (0,5 % atro, gelöst in Wasser/Aceton) oberflächlich bei 190°C nachvernetzt.

### Beispiel 2:

80 g Acrylsäure und 0,2 g eines Triacrylates von einem oxethylierten (3 Mole Ethylenoxid) Trimethylolpropan werden in 244 g Wasser gelöst und mit 8,9 g 50 %-iger Natronlauge vorneutralisiert. Nach Kühlung mit Eis auf 6 bis 8 °C und Spülung mit Stickstoff bis auf einen restlichen Sauerstoffgehalt von max. 0,3 ppm werden 0,033 g 2,2'-Azobis(2-methyl-propionamidin)-dihydrochlorid, 0,12 g Natriumpersulfat und 0,023 g Wasserstoffperoxid (35 %) eingetragen. Dann wird die Polymerisation mit 0,003 g Ascorbinsäure gestartet.
Nach ca.20 min. wird die Maximaltemperatur von 74°C erreicht. Für die Nachreaktion werden weitere 15 min. eingehalten und sodann das entstandene Polymergel durch einen Fleischwolf gegeben. Die zerkleinerte Masse wird anschließend in einer Trommel mit horizontaler Rührwelle mit 53,3 g 50 %-iger Natronlauge vermischt (Erhöhung des Neutralisationsgrades auf 70 %), bei 140 bis 160°C auf einen Wassergehalt von <10 % getrocknet und auf eine Partikelgröße von >180 bis <850 µm gemahlen. Das erhaltene Granulat wird mit Ethylencarbonat (0,5 % atro, gelöst in Wasser/Aceton) oberflächlich bei 190°C nachvernetzt.

### Beispiel 3:

80 g Acrylsäure und 0,16 g Trimethylolpropan werden in 244 g Wasser gelöst und mit 8,9 g 50 %-iger Natronlauge vorneutralisiert. Nach Kühlung mit Eis auf 6 bis 8 °C und Spülung mit Stickstoff bis auf einen restlichen Sauerstoffgehalt von max. 0,3 ppm werden 0,033 g 2'-Azobis(2-methyl-propionamidin)-dihydrochlorid, 0,12 g Natriumpersulfat und 0,023 g Wasserstoffperoxid (35 %) eingetragen. Dann wird die Polymerisation mit 0,003 g Ascorbinsäure gestartet.
Nach ca.20 min. wird die Maximaltemperatur von 74°C erreicht. Für die Nachreaktion werden weitere 15 min. eingehalten und sodann das entstandene Polymergel durch einen Fleischwolf gegeben. Die zerkleinerte Masse wird anschließend in einer Trommel mit horizontaler Rührwelle mit 53,3 g 50 %-iger Natronlauge vermischt (Erhöhung des Neutralisationsgrades auf 70 %), bei 140 bis 160°C auf einen Wassergehalt von <10 % getrocknet und auf eine Partikelgröße von >180 bis <850 µm gemahlen. Das erhaltene Granulat wird mit Ethylencarbonat (0,5 % atro, gelöst in Wasser/Aceton) oberflächlich bei 190°C nachvernetzt.

### Beispiel 4:

80 g Acrylsäure und 0,24 g eines Triacrylates von einem oxethylierten (15 Mole Ethylenoxid) Trimethylolpropan werden in 244 g Wasser gelöst und mit 8,9 g 50 %-iger Natronlauge vorneutralisiert. Nach Kühlung mit Eis auf 6 bis 8 °C und Spülung mit Stickstoff bis auf einen restlichen Sauerstoffgehalt von max. 0,3 ppm werden 0,033 g 2,2'-Azobis(2-methyl-propionamidin)-dihydrochlorid, 0,12 g Natriumpersulfat und 0,023 g Wasserstoffperoxid (35 %) eingetragen. Dann wird die Polymerisation mit 0,003 g Ascorbinsäure gestartet.
Nach ca.20 min. wird die Maximaltemperatur von 74°C erreicht. Für die Nachreaktion werden weitere 15 min. eingehalten und sodann das entstandene Polymergel durch einen Fleischwolf gegeben. Die zerkleinerte Masse wird anschließend in einer Trommel mit horizontaler Rührwelle mit 53,3 g 50 %-iger Natronlauge vermischt (Erhöhung des Neutralisationsgrades auf 70 %), bei 140 bis 160°C auf einen Wassergehalt von <10 % getrocknet und auf eine Partikelgröße von >180 bis <850 µm gemahlen. Das erhaltene Granulat wird mit Ethylencarbonat (0,5 % atro, gelöst in Wasser/Aceton) oberflächlich bei 190°C nachvernetzt.

### Beispiel 5:

80 g Acrylsäure und 1 g Polyethylen(600)-glykoldiacrylat werden zusammen mit 1 g Mowiol 5/88 (Fa.Hoechst) in 240 g Wasser gelöst und mit 8,9 g 50 %-iger Natronlauge vorneutralisiert. Nach Kühlung mit Eis auf 6 bis 8 °C und Spülung mit Stickstoff bis auf einen restlichen Sauerstoffgehalt von max. 0,3 ppm werden 0,23 g 2,2'-Azobis(2-methyl-propionamidin)-dihydrochlorid und 0,24 g Wasserstoffperoxid (35 %), eingetragen. Dann wird die Polymerisation mit 0,02 g Ascorbinsäure gestartet.
Die Maximaltemperatur von 75°C wird nach ca. 15 min. erreicht. Nach 15-minütiger Wartezeit für die Nachreaktion wird das entstandene Polymergel durch einen Fleischwolf gegeben. Die zerkleinerte Masse wird anschließend in einer Trommel mit horizontaler Rührwelle mit 190,7 g uncalcinierter, fester Soda vermischt (Erhöhung des Neutralisationsgrades auf 70 %), bei 140 bis 160°C auf einen Wassergehalt von <10 % getrocknet und auf eine Partikelgröße von >180 bis <850 µm gemahlen. Das erhaltene Granulat wird mit Ethylencarbonat (0,5 % atro, gelöst in Wasser/Aceton) oberflächlich bei 190°C nachvernetzt.

### Beispiel 6:

80 g Acrylsäure und 1 g Polyethylen(400)-glykoldiacrylat werden zusammen mit 1 g Mowiol 5/88 (Fa.Hoechst) in 240 g Wasser gelöst und mit 26,7 g 50 %-iger Natronlauge vorneutralisiert. Nach Kühlung mit Eis auf 6 bis 8 °C und Spülung mit Stickstoff bis auf einen restlichen Sauerstoffgehalt von max. 0,3 ppm werden 0,23 g 2,2'-Azobis(2-methyl-propionamidin)-dihydrochlorid und 0,24 g Wasserstoffperoxid (35 %) eingetragen. Dann wird die Polymerisation mit 0,02 g Ascorbinsäure gestartet.
Die Maximaltemperatur von 75°C wird nach ca. 15 min. erreicht. Nach 20-minütiger Wartezeit für die Nachreaktion erfolgte die Weiterverarbeitung wie in Beispiel 1 beschrieben, wobei zur Nachneutralisation 35,5 g 50 %-ige Natronlauge benutzt wurden.

### Beispiel 7:

80 g Acrylsäure und 1 g Polyethylen(400)-glykoldiacrylat werden zusammen mit 1 g Mowiol 5/88 (Fa.Hoechst) in 240 g Wasser gelöst und mit 4,5 g 50 %-iger Natronlauge vorneutralisiert. Nach Kühlung mit Eis auf 6 bis 8 °C und Spülung mit Stickstoff bis auf einen restlichen Sauerstoffgehalt von max. 0,3 ppm werden 0,23 g 2,2'-Azobis(2-methyl-propionamidin)-dihydrochlorid und 0,24 g Wasserstoffperoxid (35 %) eingetragen. Dann wird die Polymerisation mit 0,02 g Ascorbinsäure gestartet. Die Maximaltemperatur von 72°C wurde nach 25 min. erreicht. Nach 20-minütiger Wartezeit für die Nachreaktion erfolgte die Weiterverarbeitung wie in Beispiel 1 beschrieben, wobei zur Nachneutralisation 39,9 g 50 %-ige Natronlauge benutzt wurden.

### Vergleichsbeispiel 1:

### Polymerisationsversuch ohne Vorneutralisationsgrad:

80 g Acrylsäure und 0,24 g eines Triacrylates von einem oxethylierten (3 Mole Ethylenoxid) Trimethylolpropan wurden in 244 g Wasser gelöst. Nach Kühlung mit Eis auf 6 bis 8 °C und Spülung mit Stickstoff bis auf einen restlichen Sauerstoffgehalt von max. 0,3 ppm wurden 0,033 g 2,2'-Azobis(2-methyl-propionamidin)-dihydrochlorid, 0,12 g Natriumpersulfat und 0,023 g Wasserstoffperoxid (35 %) eingetragen. Dann wurde versucht, die Polymerisation (wie in Beispiel 1) mit 0,003 g Ascorbinsäure zu starten. Da dies nicht gelang, wurden 0,02 g Ascorbinsäure nachgesetzt. Nach einer Wartezeit von 5 min. sprang die Polymerisation an und erreichte nach ca. 30 min. die Maximaltemperatur von 64°C. Für die Nachreaktion wurden weitere 15 min. eingehalten und sodann das entstandene Polymergel durch einen Fleischwolf gegeben. Die zerkleinerte Masse wurde anschließend in einer Trommel mit horizontaler Rührwelle mit 62,2 g Natronlauge vermischt (Erhöhung des Neutralisationsgrades auf 70 %), bei 140 bis 160°C auf einen Wassergehalt von <10 % getrocknet und auf eine Partikelgröße von >180 bis <850 um gemahlen. Das erhaltene Granulat wurde mit Ethylencarbonat (0,5 % atro, gelöst in Wasser/Aceton) oberflächlich bei 190°C nachvernetzt.

**Tabelle 1**

| Eigenschaften der Polymerisate | | | | | | |
|---|---|---|---|---|---|---|
| | TB (g/g) | AAP_{0,3} (g/g) | AAP_{0,7} (g/g) | QD 20/120 min (g) | LA Gew.% | Rewet (g) |
| Bspl.1 | 34 | 36 | 28 | 850/900 | 4,5 | 0,5 |
| Bspl.2 | 35 | 36 | 29 | 880/920 | 5 | 1,0 |
| Bspl.3 | 35 | 36 | 28 | 850/890 | 5 | 1,0 |
| Bspl.4 | 35 | 36 | 29 | 860/920 | 5 | 1,0 |
| Bspl.5 | 34 | 37 | 30 | 1040/940 | 4 | 0,2 |
| Bspl.6 | 34 | 37 | 30 | 1120/925 | 5 | 1,0 |
| Bspl.7 | 35 | 36 | 29 | 910/920 | 4 | 2,0 |
| Vergl.Bsp.1 | 32 | 34 | 25 | 820/850 | 4 | 2,5 |
| Sanwet® IM 4000* | 31 | 29 | 19 | 577/496 | 5,2 | 2,5 |
| Sanwet® IM 7000* | 35 | 29,5 | 16 | 609/522 | 5,4 | 2,9 |
| Sanwet® IM 7000* | 33 | 34 | 20 | 529/567 | 2,8 | 8,5 |

| | | | | | | |
|---|---|---|---|---|---|---|
| * Fa. Hoechst AG | | | | | | |

**Tabelle 2**

| Retentionen saurer Polymergele (ca. 24 % wS. Acrylsäure-bez., nach Beispiel 2 bzw Vergl.Bsp. 1 mit unterschiedlichem Neutralisationsgrad: | | |
|---|---|---|
| Beispiel | Neutralisationsgrad (%) | TB (g/g) |
| Vergl.Bsp. 1 | 0 | 2,04 |
| 2 | 5 | 2.25 |
| 2 | 10 | 2,80 |
| 2 | 20 | 4,96 |
| 2 | 30 | 8,90 |

Die Daten der Tabelle 2 zeigen die starke Zunahme der Aufnahmefähigkeit von Acrylatgelen für wässrige Flüssigkeiten im Neutralisationsbereich von 0 bis 30 % der acrylsauren Polymergele anhand der Retentionswerte von physiologischer Kochsalzlösung.

## Patentansprüche

1. Pulverförmiges, Wasser- und wässrige Flüssigkeiten absorbierendes vernetztes Polymerisat, gebildet aus
a) 55 - 99,9 Gew.% mindestens eines ungesättigten, polymerisierbaren, Säuregruppen enthaltenden Monomeren, das zu 5 - 30 Mol % neutralisiert als Salz vorliegt,
b) 0 - 40 Gew.% ungesättigten, mit dem Monomeren a) copolymerisierbaren Monomeren,
c) 0,01 - 5,0 Gew.% mindestens eines Vernetzungsmittels und
d) 0 - 30 Gew.% eines wasserlöslichen Polymeren,
- wobei die Summe der Komponenten a) - d) 100 Gew.% ergibt,
**dadurch gekennzeichnet, daß** der die Säuregruppen enthaltende Anteil der polymerisierten Monomeren a) im Polymerisat zu mindestens 50 Mol % neutralisiert ist, das Polymerisat zusätzlich auf der Oberfläche nachvernetzt ist und
- eine Retention für eine 0,9 %ige wässrige NaCl-Lösung von mindestens 25g/g Polymerisat,
- eine Flüssigkeitsaufnahme unter einem Druck von 50 g/cm² von mindestens 25g/g Polymerisat,
- einen Quelldruck nach 20 Minuten von mindestens 700 g, bezogen auf 1 g Polymerisat,
- einen löslichen Anteil nach 16 Stunden von höchstens 3,5 - 10 Gew.% und
- einen Rewet von höchstens 2,0 g aufweist,
wobei die Retention, die Flüssigkeitsaufnahme, der Quelldruck, der lösliche Anteil und der Rewet wie in der Beschreibung aufgeführt, definiert sind.

2. Polymerisat nach Anspruch 1 **dadurch gekennzeichnet, daß** es eine Retention für eine 0,9 %ige, wässrige NaCl-Lösung von mindestens 30 g/g Polymerisat, eine Flüssigkeitsaufnahme unter einem Druck von 50 g/cm² von mindestens 27 g/g Polymerisat und einen Quelldruck nach 20 Minuten von mindestens 850 g, bezogen auf 1 g Polymerisat aufweist.

3. Polymerisat nach Anspruch 1 **dadurch gekennzeichnet, daß** es eine Retention für eine 0,9 %ige, wässrige NaCl-Lösung von mindestens 33 g/g Polymerisat, und einen Quelldruck nach 20 Minuten von mindestens 900 g, bezogen auf 1 g Polymerisat aufweist.

4. Polymerisat nach einem der Ansprüche 1 oder 2 **dadurch gekennzeichnet, daß** es einen Quelldruck nach 20 Minuten von mindestens 800 g und nach 2 Stunden einen Druckabfall von höchstens 20 %, bezogen auf den Wert nach 20 Minuten aufweist.

5. Polymerisat nach einem der Ansprüche 1 - 4 **dadurch gekennzeichnet, daß** es einen extrahierbaren Anteil nach 16 Stunden von höchstens 3,5 Gew.% aufweist.

6. Polymerisat nach einem der Ansprüche 1 - 5 **dadurch gekennzeichnet, daß** das wasserlösliche Polymer ein synthetisches Polymer oder Copolymer und/oder ein natürliches Polymer und/oder ein Derivat eines natürlichen Polymers ist.

7. Verfahren zur Herstellung eines Polymerisats nach den Ansprüchen 1 - 6 **dadurch gekennzeichnet, daß** eine wässrige Lösung der Komponenten a) - d) hergestellt wird, die einen Gehalt von höchstens 30 Gew.% Monomere, bezogen auf die gesamte Lösung, aufweist, die ungesättigten polymerisierbaren Säuregruppen enthaltenden Monomere a) vor der Polymerisation mit Basen zu 5,0 - 30 Mol %, neutralisiert werden, die radikalische Polymerisation bei einer Temperatur im Bereich von 5 - 30 °C, durch chemische Initiatoren und/oder durch UV-Bestrahlung ausgelöst und adiabatisch fortgeführt wird, daß Polymerisatgel nach der Grobzerkleinerung einer Feinzerkleinerung und mit Basen so neutralisiert wird, daß das Polymerisat einen Gehalt von polymerisierten, neutralisierten Säuregruppen enthaltenden Monomereneinheiten von mindestens 50 Mol % enthält, eine Trocknung des Polymerisatgels auf einen Wassergehalt von 5 - 20 Gew.%, erfolgt und das getrocknete und gemahlene Polymerisat mit mindestens einem zweifach oder mehrfachfunktionellen, mit Säuregruppen reaktionsfähigen Vernetzungsmittel bei einer Temperatur im Bereich von 140 - 240 °C zur Reaktion gebracht wird.

8. Verfahren nach Anspruch 7 **dadurch gekennzeichnet, daß** die Monomeren a) zu 10-20 Mol % neutralisiert werden, die radikalische Polymerisation bei Temperaturen von 8 - 15 °C und die Trocknung des Polymerisatgels auf einem Wassergehalt von < 10 Gew.% erfolgt.

9. Verfahren nach Anspruch 8, **dadurch gekennzeichnet, daß** als ungesättigte polymerisierbare Säuregruppen enthaltende Monomere a) olefinisch ungesättigte, polymerisierbare Mono- und/oder Dicarbonsäuren, verwendet werden.

10. Verfahren nach Anspruch 9, **dadurch gekennzeichnet, daß** als ungesättigtes polymerisierbares Säuregruppen enthaltendes Monomeres a) Acrylsäure verwendet wird.

11. Verfahren nach Anspruch 7 - 10, **dadurch gekennzeichnet, daß** neben den Säuregruppen enthaltenden Monomeren a) andere wasserlösliche ungesättigte, polymerisierbare Monomere b) verwendet werden.

12. Verfahren nach Anspruch 11, **dadurch gekennzeichnet, daß** als andere wasserlösliche ungesättigte, polymerisierbare Monomere b) Acrylamid, Methacrylamid, N-alkylierte (Meth)acrylamide, N-Methylol(meth)acrylamid, N-Vinylamide, N-Vinylformamid, N-Vinylacetamid und N-Vinyl-N-Methylacetamid, N-Vinyl-N-Methylformamid, Vinylpyrrolidon sowie Hydroxyalkyl(meth)acrylate, wie Hydroxyethylacrylat und (Meth)acrylsäureester von Polyethylenglycolmonoallylether und Allylether von Polyethylenglycolen verwendet werden.

13. Verfahren nach den Ansprüchen 7 - 12, **dadurch gekennzeichnet, daß** wasserunlösliche oder in Wasser begrenzt lösliche Monomere b), eingesetzt werden.

14. Verfahren nach Anspruch 13, **dadurch gekennzeichnet, daß** als wasserunlösliche oder in Wasser begrenzt lösliche Monomere b) Acrylsäure- und Methacrylsäureester, wie Ethylacrylat und Methylacrylat, Vinylacetat und Styrol mit einem Anteil von bis zu 10 Gew.%, bezogen auf die Menge aller Monomeren eingesetzt werden.

15. Verfahren nach den Ansprüchen 7 - 14, **dadurch gekennzeichnet, daß** die Feinzerkleinerung unter Verwendung eines Extruders mit entständiger Lochscheibe erfolgt, wobei die Öffnungen der Lochscheibe einen Durchmesser im Bereich von 2-20 mm aufweisen.

16. Verfahren nach Anspruch 15, **dadurch gekennzeichnet, daß** die Öffnungen der Lochscheibe einen Durchmesser von 5 - 15 mm aufweisen.

17. Verwendung des Polymerisates nach den Ansprüchen 1 - 6 als Komponente in Körperflüssigkeiten absorbierenden Sanitärartikeln und in Wundabdeckungen.

18. Verwendung des Polymerisates nach den Ansprüchen 1 - 6 als Komponente in Windeln, Monatsbinden und Inkontinenzartikeln.

19. Verwendung des Polymerisates nach den Ansprüchen 1 - 6 in Windeln mit einem Gewichtsanteil der Polymerisate nach den Ansprüchen 1 - 6, bezogen auf die Gewichtsmenge von Polymerisat und Fluff von 15 - 100 Gew.%.

20. Verwendung des Polymerisates nach den Ansprüchen 1 - 6 in Windeln mit einem Gewichtsanteil der Polymerisate nach den Ansprüchen 1 - 6, bezogen auf die Gewichtsmenge von Polymerisat und Fluff von 35 - 100 Gew.%, vorzugsweise von 30 - 70 Gew.%.

21. Verwendung des Polymerisates nach einem der Ansprüche 1 - 6 als Komponente in strom- und lichtleitenden Kabeln.

22. Verwendung des Polymerisates nach einem der Ansprüche 1 - 6 als Komponente in Bodenverbesserungsmitteln.

23. Verwendung des Polymerisates nach einem der Ansprüche 1 - 6 als Komponente in künstlichem Boden zur Pflanzenzüchtung.

24. Verwendung des Polymerisates nach einem der Ansprüche 1 - 6 als Komponente in Verpackungsmaterialien.

25. Verwendung des Polymerisates nach einem der Ansprüche 1 - 6 als Komponente in Depotmaterialien zur kontrollierten Abgabe von Wirkstoffen.

## Claims

1. A powdery, water and aqueous liquids absorbing cross-linked polymer formed from
a) 55-99,9 wt-% of at least one unsaturated, polymerizable, acid groups-containing monomer which is present as a salt neutralized to the extent of 5-30 mol%,
b) 0-40 wt-% of unsaturated monomers, copolymerizable with the monomer according to a),
c) 0,01-5,0 wt-% of at least one cross-linking agent and
d) 0-30 wt-% of a water-soluble polymer,
- wherein the sum of the components a)-d) amounts to 100 wt-%, **characterized in that** the portion of the polymerized acid group containing monomers a) in the polymer is neutralized to the extent of at least 50 mol %, and the polymer is subsequently cross-linked on the surface and
- has a retention for a 0,9 % aqueous NaCl-solution of at least 25 g/g polymer,
- a liquid absorption under a pressure of 50 g/cm² of at least 25 g/g polymer,
- a swelling pressure after 20 minutes of at least 700 g relative to 1 g of polymer,
- a maximum soluble portion of 3,5-10 wt-% after 16 hours and
- a maximum rewet of 2,0 g,
wherein the retention, the liquid absorption, the swelling pressure, the soluble portion and the rewet are defined as mentioned in the description.

2. The polymer according to claim 1, **characterized in that** said polymer has a retention for a 0,9 % aqueous NaCl-solution of at least 30 g/g polymer, a liquid absorption under a pressure of 50 g/cm² of at least 27 g/g polymer and a swelling pressure after 20 minutes of at least 850 g, relative to 1 g of polymer.

3. The polymer according to claim 1, **characterized in that** said polymer has a retention for a 0,9 % aqueous NaCl-solution of at least 33 g/g polymer and a swelling pressure after 20 minutes of at least 900 g relative to 1 g of polymer.

4. The polymer according to one of claims 1 or 2, **characterized in that** said polymer has a swelling pressure after 20 minutes of at least 800 g and a maximum pressure drop of 20 % after 2 hours, relative to the value after 20 minutes.

5. The polymer according to one of claims 1-4, **characterized in that** said polymer has a maximum extractable portion of 3,5 wt-% after 16 hours.

6. The polymer according to one of claims 1-5, **characterized in that** said water-soluble polymer is a synthetic polymer or copolymer and/or a natural polymer and/or a derivative of a natural polymer.

7. A method for producing a polymer according to claims 1-6, **characterized in that** an aqueous solution of the components a)-d) is formed which has a maximum monomer content of 30 wt-%, relative to the total solution, said unsaturated, polymerizable, acid group containing monomers a) are neutralized with bases to the extent of 5,0-30 mol % prior to polymerization, the radical polymerization is initiated at a temperature ranging from 5-30 °C by chemical initiators and/or by UV radiation and is continued adiabatically, after coarse comminution and comminution the polymer gel is neutralized with bases, said polymer having a content of polymerized, neutralized, acid group-containing monomer units of at least 50 mol %, a drying of the polymer gel to a content of 5-20 wt-% is effected and the dried and grinded polymer is reacted at a temperature in the range of 140-240 °C with at least one bi- or multifunctional crosslinker reactive with acid groups.

8. The method according to claim 7, **characterized in that** the monomers a) are neutralized to the extent of 10-20 mol %, the radical polymerization is effected at temperatures of 8-15 °C, and the polymer gel is dried to a water content of ≤ 10 wt-%.

9. The method according to claim 8, **characterized in that** olefinically unsaturated, polymerizable mono- and/or dicarboxylic acids are used as unsaturated, polymerizable, acid groups-containing monomers a).

10. The method according to claim 9, **characterized in that** acrylic acid is used as said unsaturated, polymerizable, acid groups-containing monomer a).

11. The method according to claim 7-10, **characterized in that** in addition to the acid groups-containing monomers a) other water-soluble, unsaturated, polymerizable monomers b) are used.

12. The method according to claim 11, **characterized in that** acrylamide, methacrylamide, N-alkylated (meth) acrylamides, N-methylol (met)acrylamide, N-vinyl amides, N-vinyl formamide, N-vinyl acetamide, and N-vinyl-N-methylacetamide, N-vinyl-N-methylformamide, vinylpyrrolidone as well as hydroxyalkyl (meth)acrylates like hydroxyethyl acrylate and (meth)acrylic acid esters of polyethylene glycol monoallyl ether, and allyl ethers of polyethylene glycols, are used as the water-soluble, unsaturated, polymerizable monomer b).

13. The method according to claims 7-12, **characterized in that** water-insoluble or sparingly water-soluble monomers b) are used.

14. The method according to claim 13, **characterized in that** acrylic acid and methacrylic acid esters like ethyl acrylate and methyl acrylate, vinyl acetate, and styrene are used as water-insoluble or sparingly water-soluble monomers b), in an amount of up to 10 wt-%, relative to the amount of all monomers.

15. The method according to claims 7-14, **characterized in that** the comminution is carried out by using an extruder having a terminal breaker plate, said terminal breaker plate having a diameter in the range of 2-20 mm.

16. The process according to claim 15, **characterized in that** the openings of the breaker plate have a diameter of 5-15 mm.

17. Use of the polymer according to claims 1-6 as component in sanitary articles and in wound covers absorbing body fluids.

18. Use of the polymer according to claims 1-6 as component in diapers, sanitary napkins and incontinence articles.

19. Use of the polymer according to claims 1-6 in diapers with a weight percentage of the polymers according to claims 1-6, relative to the combined weight of said polymer and fluff of 15-100 wt-%.

20. Use of the polymer according to claims 1-6 in diapers with a weight percentage of the polymers according to claims 1-6, relative to the combined weight of polymer and fluff of 35-100 wt-%, preferably of 30-70 wt-%.

21. Use of the polymer according to one of claims 1-6 as component in current-conducting and light-transmitting cables.

22. Use of the polymer according to one of claims 1-6 as component in soil conditioners.

23. Use of the polymer according to one of claims 1-6 as component in artificial soil for plant cultivation.

24. Use of the polymer according to one of claims 1-6 as component in packaging materials.

25. Use of the polymer according to one of claims 1-6 as component in depot materials for the controlled release of active agents.

## Revendications

1. Polymère réticulé en forme de poudre, absorbant de l'eau et des liquides aqueux, formé à partir de
a) 55 - 99,9 % en poids d'au moins un monomère insaturé polymérisable, comprenant des groupes acides, qui se présente sous forme de sel, neutralisé à 5-30 % en mole,
b) 0 - 40 % en poids de monomères insaturés, étant copolymérisables avec les monomères a),
c) 0,01 - 5,0 % en poids d'au moins un agent de réticulation et
d) 0 - 30 % en poids d'un polymère soluble dans l'eau,
- la somme des composants a) - d donnant 100 % en poids, **caractérisé en ce que** la part comportant les groupes acides des monomères polymérisés a) est neutralisée à au moins 50 % en mole dans le polymère, **en ce que** le polymère est réticulé ultérieurement sur la surface et
- qu'il présente une rétention pour une solution aqueuse de 0,9 % de NaCl d'au moins 25 g/g de polymère,
- une absorption de liquide sous une pression de 50 g/cm² d'au moins 25 g/g de polymère,
- une pression de gonflement d'au moins 700 g par rapport à 1 g de polymère après 20 minutes,
- une part soluble de 3,5-10 % en poids au maximum après 16 heures et
- un rewet de 2,0 g au maximum, la rétention, l'absorption de liquide, la pression de gonflement, la part soluble et le rewet étant définis comme mentionnés dans la description.

2. Polymère selon la revendication 1, **caractérisé en ce qu'**il présente une rétention pour une solution aqueuse de 0,9 % de NaCl d'au moins 30 g/g de polymère, une absorption de liquide sous une pression de 50 g/cm² d'au moins 27 g/g de polymère et une pression de gonflement d'au moins 850 g par rapport à 1 g de polymère après 20 minutes.

3. Polymère selon la revendication 1, **caractérisé en ce qu'**il présente une rétention pour une solution aqueuse de 0,9 % de NaCl d'au moins 33 g/g de polymère, et une pression de gonflement d'au moins 900 g par rapport à 1 g de polymère après 20 minutes.

4. Polymère selon l'une des revendications 1 ou 2, **caractérisé en ce qu'**après 20 minutes il présente une pression de gonflement d'au moins 800 g et après 2 heures une chute de pression de 20 % au maximum par rapport à la valeur après 20 minutes.

5. Polymère selon l'une des revendications 1-4, **caractérisé en ce qu'**après 16 heures il présente une part extractive de 3,5 % en poids maximum.

6. Polymère selon l'une des revendications 1-5, **caractérisé en ce que** le polymère soluble est un polymère synthétique ou un copolymère et/ou un polymère naturel et/ou un dérivé d'un polymère naturel.

7. Procédé destiné à la production d'un polymère selon les revendications 1-6, **caractérisé en ce qu'**une solution aqueuse des composants a)-d) est produite qui présente un contenu de 30 % en poids au maximum de monomères par rapport à la solution totale, que les monomères insaturés, polymérisables, contenant les groupes acides a) sont neutralisés avant la polymérisation avec des bases à 5,0-30 % en mole, que la polymérisation radicalaire est déclenchée à une température comprise entre 5-30 °C par des excitants chimiques et/ou par rayons UV et est poursuivie de façon adiabatique, qu'après le concassage grossier et un broyage fin le gel de polymère est neutralisé de telle façon que le polymère a un contenu de motifs monomères polymérisés, contenant des groupes acides neutralisés, d'au moins 50 % en mole, qu'un séchage du gel de polymère jusqu'à un contenu d'eau de 5-20 % en poids est effectué et que le polymère séché et moulu est amené à réagir avec au moins un agent de réticulation bi- ou multifonctionnel, pouvant réagir avec des groupes acides, à une température comprise entre 140-240 C.

8. Procédé selon la revendication 7, **caractérisé en ce que** les monomères a) sont neutralisés à 10-20 % en mole, que la polymérisation radicalaire est effectuée à des températures comprises entre 8-15 °C et que le séchage du gel de polymère est effectué jusqu'à un contenu d'eau de ≤ 10 % en poids.

9. Procédé selon la revendication 8, **caractérisé en ce que** comme monomères insaturés polymérisables, comportant des groupes acides a) des acides monocarboxyliques et/ou dicarboxyliques insaturés à l'oléfine et polymérisables sont utilisés.

10. Procédé selon la revendication 9, **caractérisé en ce que** comme monomère insaturé, polymérisable, comportant des groupes acides a) de l'acide acrylique est utilisé.

11. Procédé selon les revendications 7-10, **caractérisé en ce qu'**outre les monomères comportant des groupes acides a) d'autres monomères solubles insaturés, polymérisables b) sont utilisés.

12. Procédé selon la revendication 11, **caractérisé en ce que** comme d'autres monomères solubles dans l'eau, insaturés, polymérisables b) de l'acrylamide, du méthacrylamide, des (méth)acrylamides N-alkylés, du N-méthylol(méth)acrylamide, N-vinylamide, N-vinylformamide, N-vinylacétamide, et du N-vinyl-N-méthylformamide, du vinylpyrrolidone ainsi que du hydroxyalkyl(méth)acrylate comme du hydroxyethylacrylate et des esters d'acide (méth)acrylique d'éther monoallylique du polyéthylèneglycol et des éthers allyliques de polyéthylèneglycol peuvent être utilisés.

13. Procédé selon les revendications 7-12, **caractérisé en ce que** des monomères insolubles ou solubles de façon limitée dans l'eau b) sont utilisés.

14. Procédé selon la revendication 13, **caractérisé en ce que** comme monomères insolubles ou solubles de façon limitée b) de l'acrylate et de l'ester méthacrylique, comme de l'acrylate d'éthyle et du méthacrylate, de l'acétate vinylique et du styrène avec une part s'élevant jusqu'à 10 % en poids par rapport à la quantité de tous les monomères sont utilisés.

15. Procédé selon les revendications 7-14, **caractérisé en ce que** le broyage fin est effectué en utilisant une extrudeuse avec une plaque à trous d'extrémité, les ouvertures de la plaque à trous ayant un diamètre compris entre 2-20 mm.

16. Procédé selon la revendication 15, **caractérisé en ce que** les ouvertures de la plaque à trous ont un diamètre de 5-15 mm.

17. Utilisation du polymère selon les revendications 1-6, comme composant dans des articles sanitaires et dans des protections pour plaies qui absorbent des liquides corporels.

18. Utilisation du polymère selon les revendications 1-6 comme composant dans des couches, des serviettes hygiéniques et des article incontinence.

19. Utilisation du polymère selon les revendications 1-6 dans des couches avec une part de poids des polymères selon les revendications 1-6, par rapport à la quantité en poids de polymère et de « fluff» de 15-100 % en poids.

20. Utilisation du polymère selon les revendications 1 -6 dans des couches avec une part en poids des polymères selon les revendications 1-6 par rapport à la quantité en poids de polymère et de « fluff » de 35-100 % en poids, de préférence de 30-70 % en poids.

21. Utilisation du polymère selon l'une des revendications 1-6 comme composant dans des câbles conducteurs de courant et de lumière.

22. Utilisation du polymère selon l'une des revendications 1-6 comme composant dans du sol artificiel destiné à la culture de plantes.

23. Utilisation du polymère selon l'une des revendications 1-6 comme composant dans du sol artificiel pour la culture de plantes.

24. Utilisation du polymère selon l'une des revendications 1-6 comme composant dans du matériel d'emballage.

25. Utilisation du polymère selon l'une des revendications 1-6 comme composant dans des matériaux de dépôt destinés au dégagement contrôlé de substances actives.
